# EUROPÄISCHE PATENTSCHRIFT

(11) **EP 0 570 849 B1**
(45) Veröffentlichungstag und Bekanntmachung des Hinweises auf die Patenterteilung: **25.03.1998**
(21) Anmeldenummer: 93107817.4
(22) Anmeldetag: 13.05.1993
(51) Int. Cl.: C07D 501/18, C07D 501/04

(54) **Verfahren zur Spaltung von Cephalosporin Prodrugestern zu 7-Amino-3-methoxymethylceph-3-em-4-carbonsäure**
Process for the cleavage of cephalosporin prodrug esters to 7-amino-3-methoxymethylceph-3-em-4-carboxylic acid
Procédé de clivage de prodrug esters de céphalosporine en 7-amino-3-méthoxyméthylcéph-3-èm-4-carboxylique

(30) Priorität: 21.05.1992 DE 4216881
(43) Veröffentlichungstag der Anmeldung: 24.11.1993
(73) Patentinhaber: HOECHST AKTIENGESELLSCHAFT, 65926 Frankfurt am Main (DE)
(72) Erfinder: Krass, Norbert, Dr., W-6000 Frankfurt/Main 90 (DE); Defossa, Elisabeth, Dr., W-6270 Idstein/Ts. (DE); Fischer, Gerd, Dr., W-6250 Limburg 8 (DE); Gerlach, Uwe, Dr., W-6230 Frankfurt/Main 80 (DE); Hörlein, Rolf, Dr., W-6000 Frankfurt/Main 71 (DE); Lattrell, Rudolf, Dr., W-6240 Königstein/Ts. (DE); Linkies, Adolf Heinz, Dr., W-6230 Frankfurt/Main 80 (DE); Martin, Wolfgang, Dr., W-6233 Kelkheim/Ts. (DE); Stache, Ulrich, Dr., W-6238 Hofheim/Ts. (DE); Wollmann, Theodor, Dr., W-6238 Hofheim/Ts. (DE)

(56) Entgegenhaltungen:
- EP-A- 0 222 022
- CHEMICAL ABSTRACTS, vol. 82, no. 17, 28. April 1975, Columbus, Ohio, US; abstract no. 112093g, Seite 517 ;Spalte L ; & JP-A-74 108 010 (FUJISAWA)
- CHEMICAL ABSTRACTS, vol. 82, no. 5, 3. Februar 1975, Columbus, Ohio, US; abstract no. 31338p, Seite 501 ;Spalte R ; & JP-A-7 485 082 (FUJISAWA)
- CHEMICAL ABSTRACTS, vol. 98, no. 21, 23. Mai 1983, Columbus, Ohio, US; abstract no. 179032k, Seite 625 ;Spalte R ; & CHEM.PHARM.BULL. Bd. 30, Nr. 12 , 1982 , TOKYO Seiten 4545 - 4547
- JOURNAL OF THE AMERICAN CHEMICAL SOCIETY, Bd. 91, Nr. 20, 24. September 1969, WASHINGTON, Seiten 5674 - 5675; J. ALAN WEBBER ET AL.
- THE JOURNAL OF ORGANIC CHEMISTRY, Bd. 56, Nr. 11, 24. Mai 1991, WASHINGTON, Seiten 3633 - 3637, SIGERU TORII ET AL.

## Beschreibung

Die Erfindung betrifft ein Verfahren zur Herstellung der 7-Amino-3-methoxymethyl-ceph-3-em-4-carbonsäure (7-MACA) aus 7-Amino-3-methoxymethyl-ceph-3-em-4-carbonsäureestern oder deren Salzen durch Esterspaltung.

Die literaturbekannten Methoden der Esterspaltung erfordern Reaktionsbedingungen, die eine Anwendung auf 7-Amino-3-methoxymethyl-ceph-3-em-4-carbonsäureester wegen des labilen β-Lactamringes nicht in befriedigender Weise zulassen. So führt z.B. die alkalische Hydrolyse von Estern mit Basen wie KOH, NaOH oder Natriumalkoholaten in Wasser oder in organischen Lösungsmitteln wie z.B. Dioxan oder Alkohol lediglich zu Zersetzungsprodukten. Von S. Torii et al. ist die Spaltung von Cephalosporinestern mittels Phenol und Säurekatalyse beschrieben (J. Org. Chem. 56 (1991) 3633). Eine Anwendung dieses Verfahrens auf 7-Amino-3-methoxymethyl-ceph-3-em-4-carbonsäureester lieferte jedoch die gewünschte Carbonsäure lediglich in geringer Ausbeute und ungenügender Reinheit.

Für die Synthese diastereomerenreiner Cephalosporin-Prodrugester, wie sie z.B. in EP-A-0 329 008, EP-A-0 514 791 und EP-A-0 531 875 beschrieben sind, ist es essentiell, daß die 7-Amino-3-methoxymethyl-ceph-3-em-4-carbonsäureester, die nach Veresterung der entsprechenden Carbonsäuren als Diastereomerengemisch anfallen, in die reinen Diastereomeren getrennt werden. Dabei werden Diastereomere mit unterschiedlicher pharmakologischer Wirksamkeit erhalten. Der Erfindung liegt daher die Aufgabe zugrunde, ein Verfahren zu entwickeln, mit dem 7-Amino-3-methoxymethyl-ceph-3-em-4-carbonsäureester, insbesondere ein weniger aktives Diastereomer oder auch unterschiedliche Gemische desselben, mit hoher Ausbeute wieder in die entsprechenden Carbonsäuren überführt und so für eine erneute Veresterung genutzt werden können.

Diese Aufgabe wird erfindungsgemäß gelöst durch das Verfahren zur Herstellung der 7-Amino-3-methoxymethyl-ceph-3-em-4-carbonsäure der Formel I, 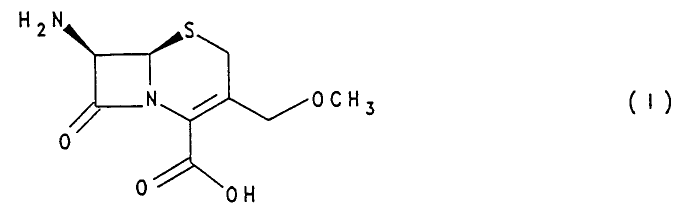 das dadurch gekennzeichnet ist, daß man 7-Amino-3-methoxymethyl-ceph-3-em-4-carbonsäureester der Formel II oder deren Salze 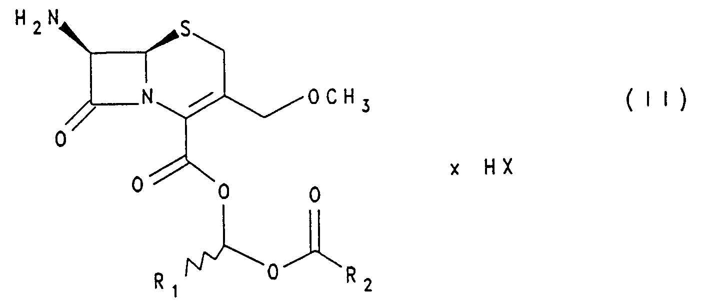 worin
R¹ für Methyl oder Ethyl,
R² für C₁-C₆-Alkyl oder C₁-C₆-Alkoxy und HX für eine- oder mehrbasische Säuren steht und HX eine organische oder anorganische Säure ist, mit einer Protonensäure oder einem Gemisch zweier Säuren, wie Ameisensäure, Trifluoressigsäure, Methansulfonsäure und Trifluormethansulfonsäure, umgesetzt werden.

In R² kann C₁-C₆-Alkyl dabei für Methyl, Ethyl, n-Propyl, Isopropyl, n-Butyl, Isobutyl, tert.-Butyl, Pentyl, Cyclopentyl, Hexyl und Cyclohexyl stehen, bevorzugt für n-Propyl, Isopropyl, n-Butyl, tert.Butyl und Cyclohexyl, insbesondere für tert.Butyl; und C₁-C₆-Alkoxy für Methoxy, Ethoxy, n-Propoxy, Isopropoxy, n-Butoxy, Isobutoxy, tert.Butoxy, n-Pentyloxy, Cyclopentyloxy, n-Hexyloxy und Cyclohexyloxy, bevorzugt für n-Propoxy, Isopropoxy, n-Butoxy, tert.Butoxy und Cyclohexyloxy, besonders bevorzugt für Isopropoxy.

Die Verbindungen der allgemeinen Formel II werden als freie Basen oder in Form ihrer Salze (mit HX) eingesetzt, wobei HX für eine ein- oder mehrbasische Säure steht und X ein anorganisches oder organisches, physiologisch unbedenkliches Anion sein kann. Als anorganische Säure bedeutet HX beispielsweise stöchiometrische Mengen an HCl, HBr, HI, HBF₄, HNO₃, HClO₄, H₂SO₄ oder H₃PO₄. Als organische Säure steht HX für aliphatische oder aromatische Sulfonsäuren und für Ameisensäure, Essigsäure und Trifluoressigsäure. Bevorzugt steht HX für die anorganischen Säuren HCl, HBr und H₂SO₄ sowie die organischen Säuren Methansulfonsäure, Ethansulfonsäure, Benzolsulfonsäure, p-Toluolsulfonsäure und 4-Ethylbenzolsulfonsäure, Ameisensäure, Essigsäure und Trifluoressigsäure. Besonders bevorzugt sind die Säuren HCl, Methansulfonsäure und p-Toluolsulfonsäure.

Das erfindungsgemäße Verfahren zeichnet sich dadurch aus, daß Verbindungen der allgemeinen Formel II mit einer Protonensäure oder einem Gemisch zweier Säuren umgesetzt werden. Hierzu können folgende Säuren eingesetzt werden: Ameisensäure, Trifluoressigsäure, Methansulfonsäure und Trifluormethansulfonsäure. Bevorzugt sind Trifluoressigsäure, Methansulfonsäure und Trifluormethansulfonsäure und nachfolgende Kombinationen: Trifluoressigsäure/Methansulfonsäure, Trifluoressigsäure/Trifluormethansulfonsäure, Ameisensäure/Schwefelsäure, Ameisensäure/Trifluoressigsäure, Ameisensäure/Methansulfonsäure und Ameisensäure/Trifluormethansulfonsäure. Besonders bevorzugt sind Ameisensäure/Schwefelsäure, Trifluoressigsäure, Methansulfonsäure und Trifluormethansulfonsäure oder Gemische aus jeweils zwei dieser zuletzt genannten Säuren.

Beim Einsatz eines Gemisches zweier Säuren liegen die Verhältnisse der Moläquivalente zwischen 20:1 und 1:1, vorzugsweise zwischen 12:1 und 5:1, insbesondere bei einem Verhältnis von 10:1.

Bezogen auf den eingesetzten Cephalosporinester kann die verwendete Menge an Säure zwischen 1 und 20, vorzugsweise zwischen 8 und 12, insbesondere bei 11 Moläquivalenten liegen. Wird als Lösungsmittel Hexafluorisopropyl eingesetzt, kann die verwendete Menge an Saure zwischen 1 und 2, bevorzugt zwischen 1 und 1,5 Moläquivalenten liegen. Falls kein gesondertes Lösungsmittel verwendet wird, liegt der Anteil Cephalosporinester:Säure jedoch zwischen 40 und 80, bevorzugt zwischen 50 und 60 Moläquivalenten.

Die Reaktion des erfindungsgemäßen Verfahrens kann wahlweise lösungsmittelfrei oder unter Zusatz eines der folgenden Lösungsmittel durchgeführt werden: Wasser, Aceton, Tetrahydrofuran, Dioxan, Diethylether, Acetonitril, Dichlormethan, Benzol, Toluol, Anisol und 1,1,1,3,3,3-Hexafluorisopropanol (HFIP). Besonders bevorzugt sind Dichlormethan, Hexafluorisopropanol, Acetonitril und Toluol, insbesondere Dichlormethan und Hexafluorisopropanol.

Die Reaktionstemperatur liegt je nach Lösungsmittel zwischen etwa -10°C und +40°C, vorzugsweise zwischen 0°C und 25°C (Raumtemperatur). Bevorzugt wird die Reaktion bei Raumtemperatur durchgeführt, falls CH₂Cl₂ als Lösungsmittel und zwischen 0°C und +5°C, falls HFIP als Lösungsmittel verwendet wird. Die Reaktionszeit kann je nach Reaktionstemperatur, Lösungsmittel und Säureadditionsprodukt zwischen 2 und 20 Stunden betragen.

Die Aufarbeitung und Isolierung der nach dem erfindungsgemäßen Verfahren hergestellten 7-Amino-methoxymethyl-ceph-3-em-4-carbonsäure (7-MACA) der Formel II erfolgt nach Hydrolyse mit Eiswasser und Fällung durch Zugabe von Base, wie z.B. Ammoniak, 10-40%iger Kali- oder Natronlauge. Das Produkt wird mit Wasser, Aceton und Diethylether gewaschen und in üblicher Weise getrocknet. Verunreinigungen können wahlweise durch Versetzen der Produktlösung mit Aktivkohle bzw. durch Ausrühren oder durch Chromatographie über ®Diaion HP 20 (Mitsubishi Chem. Ind., Ltd.) entfernt werden.

Das erfindungsgemäße Verfahren zeichnet sich gegenüber anderen, literaturbekannten Esterhydrolyse-Methoden dadurch aus, daß es die Verbindung der Formel I in guter Ausbeute und sehr hoher Reinheit liefert. Das so erhaltene Produkt wird durch Veresterung analog der Beschreibung in EP-A-0 329 008, EP-A-0 514 791 und EP-A-0 531 875 in Gemische der allgemeinen Formel II überführt, wobei sich die durch das erfindungsgemäße Verfahren rezyklisierte Verbindung der Formel I durch gleiches Reaktionsverhalten wie die kommerziell erhältliche 7-MACA der Firma Biochemie (Kundl, Österreich) auszeichnet.

### Beispiel 1

80 g (195.6 g mmol) 7-Amino-3-methoxymethyl-ceph-3-em-4-carbonsäure-1-(2,2-dimethyl-propionyloxy)-ethylester-Hydrochlorid werden in 400 ml Methylenchlorid suspendiert und mit 222,4 g (1.95 mol) Trifluoressigsäure und 18.8 g (195.6 mmol) Methansulfonsäure versetzt. Nach 2 Stunden Rühren bei Raumtemperatur engt man den Reaktionsansatz im Vakuum ein und nimmt den öligen Rückstand in 120 ml Wasser auf. Die Lösung wird 10 Minuten über 160 g ®Diaion HP 20 (Mitsubishi Chem. Ind., Ltd.) ausgerührt, der Feststoff über eine Nutsche abgesaugt und mit 400 ml Wasser gewaschen. Das Filtrat wird mit 10 g ®Clarocarbon F (Merck 2508) behandelt, die Aktivkohle über Filterschichten abgetrennt und mit 40 ml Wasser gewaschen. Das Produkt wird durch Zugabe von konz. NH₄OH unter Eiskühlung bei einem pH-Wert von 2.5 ausgefällt. Der Feststoff wird abfiltriert, je zweimal mit 100 ml Wasser, Aceton und Ether gewaschen und im Ölpumpenvakuum getrocknet.
Ausbeute: 27 g (57 %) 7-Amino-3-methoxymethyl-3-ceph-em-4-carbonsäure (7-MACA).
¹H-NMR (270 MHz, DMSO-d₆): δ = 3.20 (s,3H,OCH₃); 3.35 - 3.60 (AB-System, 2H, SCH₂); 4.15 (s,2H,OCH₂); 4.76 (d,1H, H-6); 4.98 (d,1H,C-7).

### Beispiel 2

5.0 g (9.2 mmol) des p-Toluolsulfonates des 7-Amino-3-methoxymethyl-ceph-3-em-4-carbonsäure-1-(2,2-dimethylpropionyloxy)ethylesters in 20 ml Methylenchlorid werden mit 10.46 g (91.7 mmol) Trifluoressigsäure versetzt und 16 Stunden bei Raumtemperatur gerührt. Nach Einengen des Reaktionsansatzes im Vakuum wird der Rückstand in Wasser aufgenommen, über Aktivkohle filtriert und über 10 g ®Diaion HP 20 (Mitsubishi Chem. Ind., Ltd.) mit 2 n Salzsäure als Laufmittel chromatographiert. Die vereinigten Produktfraktionen engt man im Vakuum auf die Hälfte des ursprünglichen Volumens ein und stellt den pH-Wert der Lösung durch Zugabe von konz. NH₄OH unter Eiskühlung auf einen Wert von 2.5 ein. Nach 30 Minuten bei 0°C wird die ausgefallene 7-MACA abgesaugt und nacheinander mit Wasser, Aceton und Diethylether gewaschen.
Ausbeute: 1.4 g (62 %) weißer Feststoff, identisch mit dem Produkt aus Beispiel 1.

### Beispiel 3

Eine Lösung von 4.91 (9.0 mmol) 7-Amino-3-methoxymethyl-ceph-3-em-4-carbonsäure-1-(2,2-dimethylpropionyloxy)ethylester-Toluolsulfonat werden in 20 ml Ameisensäure suspendiert und mit 0.2 ml konz. Schwefelsaure versetzt. Nach 3 Stunden Rühren bei Raumtemperatur filtriert man die Reaktionslösung über ®Diaion HP 20 und eluiert mit 2N HCl. Die anschließende Isolierung der 7-MACA erfolgt analog Beispiel 2.
Ausbeute: 590 mg (27 %).

### Beispiel 4

4.3 g (10.5 mmol) 7-Amino-3-methoxymethyl-ceph-3-em-4-carbonsäure-1-(2,2-dimethylpropionyloxy)ethylester-Hydrochlorid werden in 50 ml Hexafluorisopropanol gelöst und mit 0.8 ml (12.3 mmol) Methansulfonsäure versetzt. Nach 17 Stunden bei 0-5°C gibt man 20 g Eis zu dem Reaktionsansatz und stellt den pH-Wert mit konz. NH₄OH auf einen Wert von 2.5 ein. Hexafluorisopropanol wird bei Raumtemperatur im Vakuum abdestilliert, der pH-Wert erneut auf 2.5 eingestellt und die wäßrige Suspension zur Vervollständigung der Fällung weitere 60 Minuten bei 0°C gerührt. Der Feststoff wird abgesaugt und mit Eiswasser, Methanol und Aceton gewaschen. Das Rohprodukt wird in einer Salzsäure/Eiswasser-Mischung gelöst und durch Zugabe von Aktivkohle (®Clarocarbon F, Merck 2508) gereinigt. Nach Filtration kann die 7-Amino-3-methoxymethyl-ceph-3-em-4-carbonsäure wie oben beschrieben erneut ausgefällt werden.
Ausbeute: 1.9 g (74 %) weißer Feststoff.

### Beispiel 5

10 g (24.4 mmol) 7-Amino-3-methoxymethyl-ceph-3-em-4-carbonsäure-1-(2,2-dimethylpropionyloxy)ethylester-Hydrochlorid werden in einer Lösung von 5 g Natriumbicarbonat in 60 ml Wasser suspendiert, mit 100 ml Ethylacetat versetzt und 20 Minuten bei Raumtemperatur gerührt. Die Phasen werden getrennt und die wäßrige Phase zweimal mit je 50 ml Ethylacetat extrahiert. Die vereinigten organischen Phasen werden im Vakuum eingeengt und der Rückstand mit 4.66 g (24.5 mmol) p-Toluolsulfonsäurehydrat versetzt. Nach 1 Minute gibt man zu dem Reaktionsansatz 100 ml Trifluoressigsäure und rührt 3 Stunden bei Raumtemperatur. Die weitere Durchführung erfolgt wie im Beispiel 4 beschrieben.
Ausbeute: 4.08 g (70 %) farblose Kristalle.

### Vergleichsbeispiel

Analog Torii et. al. (J. Org. Chem. 56 (1991) 3633) wird eine Suspension von 1.09 g (2.0 mmol) 7-Amino-3-methoxymethyl-ceph-3-em-4-carbonsäure-1-(2,2-dimethylproprionyloxy)ethylester-Tosylat in 4 ml Acetonitril mit 0.94 g (10 mmol) Phenol und 1.154 ml (2.0 mmol) Trifluoressigsäure versetzt und bei 40°C gerührt. Die Reaktion wird per HPLC kontrolliert (®LiChrosorb RP18, 250 x 4 mm; Hibar) Laufmittel: A = MeOH/Wasser 4:1 + 0.1 % NH₄OAc, Gradient mit Wasser (Laufmittel B) bis auf A/B = 6:4). Nach 6 Stunden hat sich das Edukt vollständig umgesetzt, wobei ein komplexes Produktgemisch entstanden ist. Referenzmessungen mittels HPLC belegen, daß die gewünschte 7-MACA in dem Gemisch lediglich mit einem relativen Anteil von 3.5 % enthalten ist.

## Patentansprüche

1. Verfahren zur Herstellung der 7-Amino-3-methoxymethyl-ceph-3-em-4-carbonsäure der Formel I, 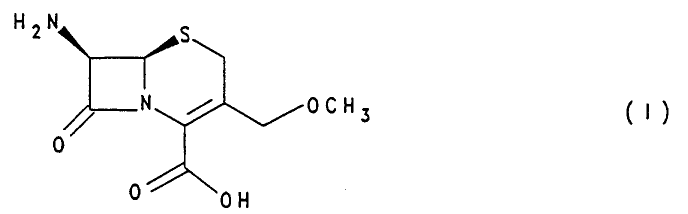 dadurch gekennzeichnet, daß man 7-Amino-3-methoxymethyl-ceph-3-em-4-carbonsäureester der Formel II oder deren Salze 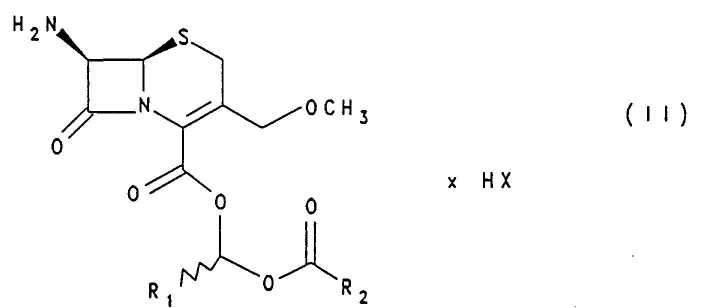 worin
R¹ für Methyl oder Ethyl,
R² für C₁-C₆-Alkyl oder C₁-C₆-Alkoxy und HX für eine- oder mehrbasische Säuren steht und HX eine organische oder anorganische Säure ist, mit einer Protonensäure oder einem Gemisch zweier Säuren, wie Ameisensäure, Trifluoressigsäure, Methansulfonsäure und Trifluormethansulfonsäure, umgesetzt werden.

2. Verfahren zur Herstellung der 7-Amino-3-methoxymethyl-ceph-3-em-4-carbonsäure der Formel I gemäß Anspruch 1, dadurch gekennzeichnet, daß
R¹ Methyl oder Ethyl,
R² n-Propyl, Isopropyl, n-Butyl, tert.Butyl, Cyclohexyl, n-Propoxy, Isopropoxy, n-Butoxy, tert.Butoxy oder Cyclohexyloxy bedeuten und
HX für eine anorganische Säure, wie HCl, HBr, HI, HBF₄, HNO₃, HClO₄, H₂SO₄ oder H₃PO₄ oder eine organische Säure, wie aliphatische oder aromatische Sulfonsäuren und für Ameisensäure, Essigsäure und Trifluoressigsäure steht.

3. Verfahren zur Herstellung der 7-Amino-3-methoxymethyl-ceph-3-em-4-carbonsäure der Formel I gemäß Anspruch 1 oder 2, dadurch gekennzeichnet, daß Trifluoressigsäure, Methansulfonsäure und Trifluormethansulfonsäure sowie Kombinationen wie Trifluoressigsäure/Methansulfonsäure, Trifluoressigsäure/Trifluormethansulfonsäure, Ameisensäure/Schwefelsäure, Ameisensäure/Trifluoressigsäure, Ameisensäure/Methansulfonsäure und Ameisensäure/Trifluormethansulfonsäure verwendet werden.

## Claims

1. A process for the preparation of 7-amino-3-methoxymethylceph-3-em-4-carboxylic acid of the formula I, 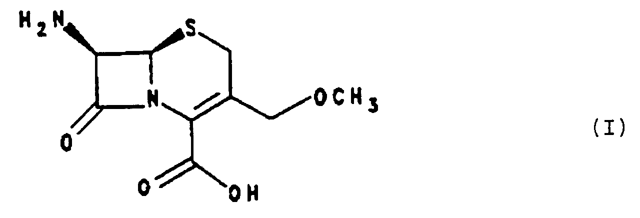 which comprises reacting 7-amino-3-methoxymethylceph-3-em-4-carboxylic acid esters of the formula II or their salts 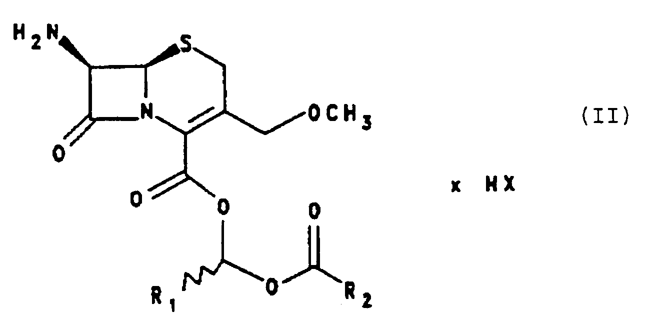 in which
R¹ is methyl or ethyl,
R² is C₁-C₆-alkyl or C₁-C₆-alkoxy and HX is a mono- or polybasic acid and HX is an organic or inorganic acid , with a protonic acid or a mixture of two acids, such as formic acid, trifluoroacetic acid, methanesulfonic acid and trifluoromethanesulfonic acid.

2. The process for the preparation of 7-amino-3-methoxymethylceph-3-em-4-carboxylic acid of the formula I as claimed in claim 1, wherein
R¹ is methyl or ethyl,
R² is n-propyl, isopropyl, n-butyl, tert-butyl, cyclohexyl, n-propoxy, isopropoxy, n-butoxy, tert-butoxy or cyclohexyloxy and
HX is an inorganic acid, such as HCl, HBr, HI, HBF₄, HNO₃, HClO₄, H₂SO₄ or H₃PO₄, or an organic acid, such as aliphatic or aromatic sulfonic acids and formic acid, acetic acid and trifluoroacetic acid.

3. The process for the preparation of 7-amino-3-methoxymethylceph-3-em-4-carboxylic acid of the formula I as claimed in claim 1 or 2, wherein trifluoroacetic acid, methanesulfonic acid and trifluoromethanesulfonic acid and combinations such as trifluoroacetic acid/methanesulfonic acid, trifluoroacetic acid/trifluoromethanesulfonic acid, formic acid/sulfuric acid, formic acid/trifluoroacetic acid, formic acid/methanesulfonic acid and formic acid/trifluoromethanesulfonic acid are used.

## Revendications

1. Procédé pour la préparation de l'acide 7-amino-3-méthoxyméthyl-céph-3-ème-4-carboxylique de formule I 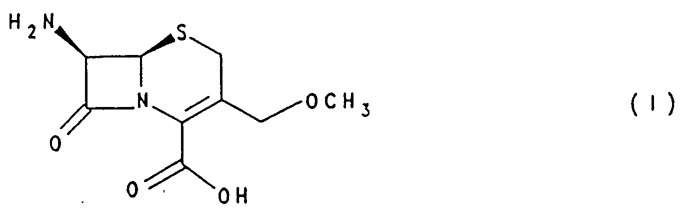 caractérisé en ce que l'on fait réagir des esters d'acide 7-amino-3-méthoxyméthyl-céph-3-ème-4-carboxylique de formule II ou leurs sels 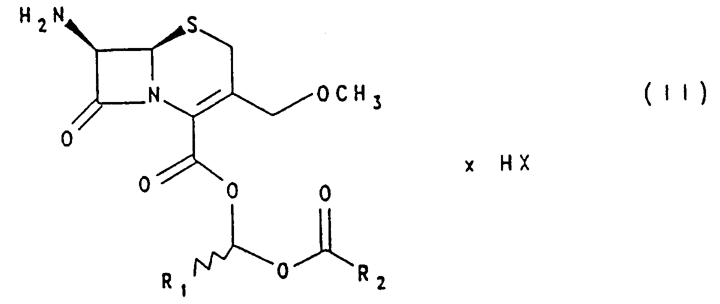 formule dans laquelle
R¹ représente le groupe méthyle ou éthyle,
R² représente un groupe alkyle en C₁-C₆ ou alcoxy en C₁-C₆, et
HX représente des mono- ou polyacides, et HX représente un acide organique ou minéral,
avec un acide protonique ou un mélange de deux acides, tels que l'acide formique, l'acide trifluoroacétique, l'acide méthanesulfonique et l'acide trifluorométhanesulfonique.

2. Procédé pour la préparation de l'acide 7-amino-3-méthoxyméthyl-céph-3-ème-4-carboxylique de formule I selon la revendication 1, caractérisé en ce que
R¹ représente le groupe méthyle ou éthyle,
R² représente le groupe n-propyle, isopropyle, n-butyle, tert-butyle, cyclohexyle, n-propoxy, isopropoxy, n-butoxy, tert-butoxy ou cyclohexyloxy, et
HX représente un acide minéral tel que HCl, HBr, HI, HBF₄, HNO₃, HClO₄, H₂SO₄ ou H₃PO₄, ou un acide organique, tel que des acides sulfoniques aliphatiques ou aromatiques, ou l'acide formique, l'acide acétique ou l'acide trifluoroacétique.

3. Procédé pour la préparation de l'acide 7-amino-3-méthoxyméthyl-céph-3-ème-4-carboxylique de formule I selon la revendication 1 ou 2, caractérisé en ce que l'on utilise l'acide trifluoroacétique, l'acide méthanesulfonique et l'acide trifluorométhanesulfonique, ainsi que des associations telles qu'acide trifluoroacétique/acide méthanesulfonique, acide trifluoroacétique/acide trifluorométhanesulfonique, acide formique/acide sulfurique, acide formique/acide trifluoroacétique, acide formique/acide méthanesulfonique et acide formique/acide trifluorométhanesulfonique.
